# EUROPEAN PATENT SPECIFICATION

(11) **EP 3 601 601 B1**
(45) Date of publication and mention of the grant of the patent: **06.11.2024**
(21) Application number: 18774651.6
(22) Date of filing: 29.03.2018
(51) Int. Cl.: G01N 33/50, C12Q 1/48

(54) **LYMPHOCYTE-BASED PKCEPSILON TEST FOR ALZHEIMER'S DISEASE**
AUF LYMPHOZYTEN BASIERTER PKCEPSILON-TEST FÜR ALZHEIMER-KRANKHEIT
TEST PKCEPSILON BASÉ SUR LES LYMPHOCYTES DÉDIÉ À LA MALADIE D'ALZHEIMER

(30) Priority: 31.03.2017 US 201762479663 P; 10.11.2017 US 201762584381 P
(43) Date of publication of application: 05.02.2020
(73) Proprietor: Neurodiagnostics LLC, Rockville, MD 20850 (US)
(72) Inventor: CHIRILA, Florin Valentin, Morgantown, WV 26501 (US); ALKON, Daniel L., Chevy Chase, MD 20815 (US)
(74) Representative: Abitz & Partner
(86) International application number: PCT/US2018/025137
(87) International publication number: WO 2018/183669

(56) References cited:
- WO-A1-2016/144838
- WO-A2-2004/076677
- US-A1- 2011 212 474
- US-A1- 2014 038 186
- US-A1- 2014 295 425
- NELSON THOMAS J ET AL: "Bryostatin Effects on Cognitive Function and PKC[epsilon] in Alzheimer's Disease Phase IIa and Expanded Access Trials", JOURNAL OF ALZHEIMER'S DISEASE, IOS PRESS, NL, vol. 58, no. 2, 1 January 2017 (2017-01-01), pages 521 - 535, XP002793126, ISSN: 1875-8908, DOI: 10.3233/JAD-170161
- KHAN ET AL.: "PKCe deficits in Alzheimer's disease brains and skin fibroblasts", J ALZHEIMERS DIS., vol. 43, no. 2, 2015, pages 491 - 509, XP055552900

## Description

This application claims the benefit of U.S. Provisional Applications No. 62/584,381, filed November 10, 2017 and 62/479,663, filed March 31, 2017.

### Field of the Invention

The present invention relates to PKCε-based methods for diagnosing Alzheimer's disease in a human subject, and for determining whether a human subject is predisposed to having Alzheimer's disease.

### Background of the Invention

Many data have been collected over the last 15 years indicating that the pathophysiology of Alzheimer's disease is not just related to the brain, but can also have systemic expression. For example, many of the critical amyloid and tau enzymes can be found throughout the body. On this basis, accurate assays have been developed to test skin cells, for example, for Alzheimer's disease against cells from a variety of control patients. Some skin cell-based assays focus on targets such as PKCε and ERK_{1,2}. US2014/038186 teaches a method of diagnosing Alzheimer's disease in a subject, wherein the level of the kinase PKC epsilon is determined in fibroblasts of the subject, and wherein a lower level of PKC epsilon in the human subject in comparison to a control subject is indicative of Alzheimer's disease. US2011/212474 which discloses a method for the diagnosis of Alzheimer's disease by using the PKC isozyme index, which relates to the expression of different PKC isozymes, such as PKC-alpha, PKC-beta, PKC-epsilon and PKC-gamma, in skin fibroblasts of a test subject in the presence or absence of beta-amyloid peptide.

### Summary of the Invention

Disclosed but not claimed is a first method, namely, a method for diagnosing Alzheimer's disease in a symptomatic human subject comprising the steps of (a) culturing lymphocytes from the subject under conditions that preserve lymphocyte viability and/or permit the lymphocytes to proliferate; (b) measuring the amount of PKCε in the cultured lymphocytes; and (c) comparing the measurement of step (b) with a suitable control, thereby determining whether the subject is afflicted with Alzheimer's disease.

Disclosed but not claimed is a second method, namely, a method for determining whether a human subject is predisposed to becoming afflicted with Alzheimer's disease comprising the steps of (a) culturing lymphocytes from the subject under conditions that preserve lymphocyte viability and/or permit the lymphocytes to proliferate; (b) measuring the amount of PKCε in the cultured lymphocytes; and (c) comparing the measurement of step (b) with a suitable control, thereby determining whether the subject is predisposed to becoming afflicted with Alzheimer's disease.

Disclosed but not claimed is a third method, namely, a method for diagnosing Alzheimer's disease in a symptomatic human subject comprising the steps of (a) culturing two otherwise identical populations of lymphocytes from the subject under conditions that preserve lymphocyte viability and/or permit the lymphocytes to proliferate, the first population comprising a suitable concentration of amylospheroid and the second population being free of amylospheroid; (b) separately measuring the amount of PKCε in the cultured lymphocytes from the first and second populations; and (c) comparing the measurements of step (b), whereby the subject is afflicted with Alzheimer's disease if the amount of PKCε in the cultured lymphocytes from the first population is greater than or equal to the amount of PKCε in the cultured lymphocytes from the second population.

Disclosed but not claimed is a fourth method, namely, a method for determining whether a human subject is predisposed to becoming afflicted with Alzheimer's disease comprising the steps of (a) culturing two otherwise identical populations of lymphocytes from the subject under conditions that preserve lymphocyte viability and/or permit the lymphocytes to proliferate, the first population comprising a suitable concentration of amylospheroid and the second population being free of amylospheroid; (b) separately measuring the amount of PKCε in the cultured lymphocytes from the first and second populations; and (c) comparing the measurements of step (b), whereby the subject is predisposed to becoming afflicted with Alzheimer's disease if the amount of PKCε in the cultured lymphocytes from the first population is greater than or equal to the amount of PKCε in the cultured lymphocytes from the second population.

Disclosed but not claimed is a fifth method, namely, a method for diagnosing Alzheimer's disease in a symptomatic human subject comprising the steps of (a) culturing two otherwise identical populations of lymphocytes from the subject under conditions that preserve lymphocyte viability and/or permit the lymphocytes to proliferate, the first population comprising a first suitable concentration of amylospheroid and the second population comprising a second suitable concentration of amylospheroid higher than the first concentration; (b) separately measuring the amount of PKCε in the cultured lymphocytes from the first and second populations; and (c) comparing the measurements of step (b), whereby the subject is afflicted with Alzheimer's disease if the amount of PKCε in the cultured lymphocytes from the second population is greater than the amount of PKCε in the cultured lymphocytes from the first population.

Finally, disclosed but not claimed is a sixth method, namely, a method for determining whether a human subject is predisposed to becoming afflicted with Alzheimer's disease comprising the steps of (a) culturing two otherwise identical populations of lymphocytes from the subject under conditions that preserve lymphocyte viability and/or permit the lymphocytes to proliferate, the first population comprising a first suitable concentration of amylospheroid and the second population comprising a second suitable concentration of amylospheroid higher than the first concentration; (b) separately measuring the amount of PKCε in the cultured lymphocytes from the first and second populations; and (c) comparing the measurements of step (b), whereby the subject is predisposed to becoming afflicted with Alzheimer's disease if the amount of PKCε in the cultured lymphocytes from the second population is greater than the amount of PKCε in the cultured lymphocytes from the first population.

### Brief Description of the Figures

### Figure 1

### Low PKCe level in blood lymphocytes

The PKCe level in an Alzheimer disease patient (AD - turquoise) is the lowest compared with the other two cases, a Huntington disease case (Non-ADD - light blue) and an Age-Matched Control (AC - purple). This result is analogous to the PKCe effect seen in skin fibroblasts and brain (Khan and Alkon, Journal of Alzheimer's Disease, 43 (2015) 491-509).

### Figures 2A and 2B

### Linear correlation of total protein concentration with B lymphocytes in the PKCe biomarker

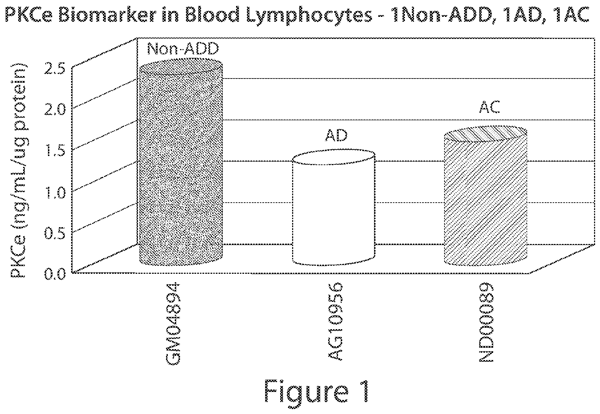

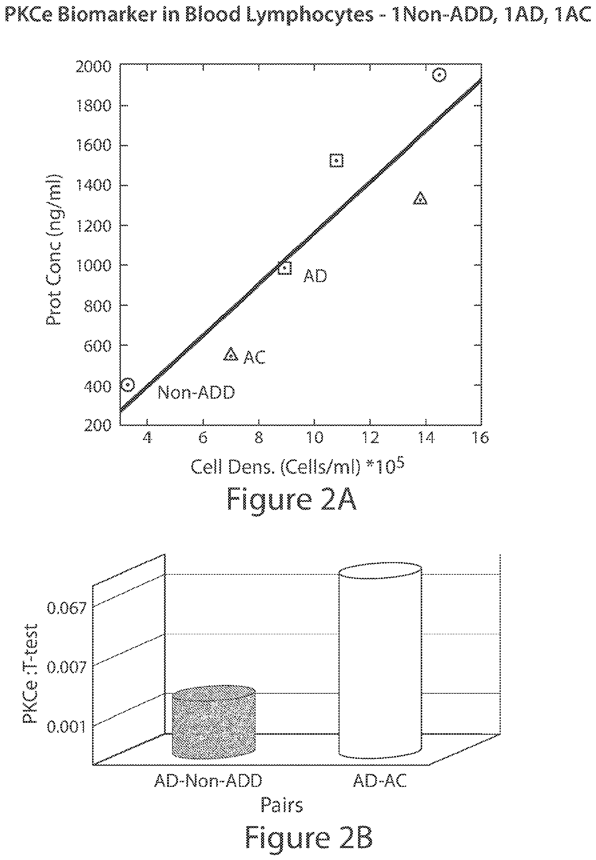

(Figure 2A) The total protein concentration for B lymphocytes correlates linearly with the cell density (Pearson's linear correlation coefficient 0.916).

(Figure 2B) The PKCe level in the AD case is lower than the Non-ADD case with a statistical significance of P<0.001 (blue cylinder). The PKCe level in the AD case is not statistically significantly lower than the AC (yellow cylinder). However, the difference between AD and AC can be assessed clinically, as the AC cases are non-demented.

### Figures 3A and 3B

### Differential PKCe values as a function of ASPD concentration

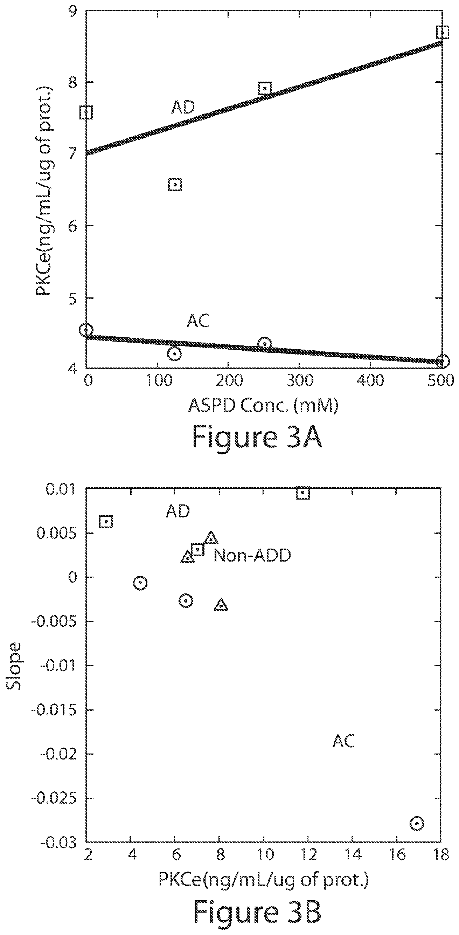

(Figure 3A) PKCe values are shown for ASPD-treated B lymphocytes for an AD case (purple squares) and an AC case (green circles). Lines represent the best fit. (Figure 3B) The distribution of the slopes (S) and intercepts (I) is shown for nine cell lines. Green circles represent AC. Purple squares represent AD. Blue triangles represent Non-ADD.

### Detailed Description of the Invention

### Definitions

In this application, certain terms are used which shall have the meanings set forth as follows.

As used herein, "diagnosing Alzheimer's disease", with respect to a symptomatic human subject, means determining that there is greater than 50% likelihood that the subject is afflicted with Alzheimer's disease. Preferably, "diagnosing Alzheimer's disease" means determining that there is greater than 60%, 70%, 80% or 90% likelihood that the subject is afflicted with Alzheimer's disease. As used herein, the phrase "determining whether the subject is afflicted with Alzheimer's disease" is synonymous with the phrase "diagnosing Alzheimer's disease."

As used herein, "determining whether a human subject is predisposed to becoming afflicted with Alzheimer's disease" means determining that there is a greater than average likelihood that the subject will become afflicted with Alzheimer's disease during her or his lifetime. After the age of 65, one in five individuals will become afflicted with Alzheimer's disease, and after the age of 85, one in two individuals will become afflicted with Alzheimer's disease. Preferably, determining this predisposition means determining that there is at least a 20% likelihood that the subject will become afflicted with Alzheimer's disease during her or his lifetime.

As used herein, "Alzheimer's disease" means a concurrent affliction with the following three symptoms: (i) dementia; (ii) amyloid plaques; and (iii) neurofibrillary tangles. Dementia can be diagnosed during life. Cerebral amyloid plaques and neurofibrillary tangles can, for example, be diagnosed during autopsy. This definition of Alzheimer's disease is the one provided by the National Institute of Neurological Disorders and Stroke (NINDS) of the National Institutes of Health (NIH), and is known as the "gold standard."

As used herein, a human subject who is "symptomatic" for Alzheimer's disease is a subject displaying at least one symptom of the disease, i.e., one of dementia, amyloid plaques, and neurofibrillary tangles. Preferably, a human subject who is symptomatic for Alzheimer's disease is a subject displaying dementia. Conversely, a human subject who is "asymptomatic" for Alzheimer's disease is a subject who does not display any symptom of the disease.

As used herein, the "human subject" can be of any age. In one embodiment, the subject is 40 years old or younger. In another embodiment, the subject is 50 years old or younger. In a further embodiment, the subject is over 40 years old. In yet a further embodiment, the subject is over 50 years old, over 60 years old, over 70 years old, over 80 years old, or over 90 years old.

As used herein, "culturing" lymphocytes under conditions permitting them to "proliferate" is achieved, for example, by conducting the culturing at a temperature and in a growth factor milieu permissive of cell growth. In another embodiment, "culturing" lymphocytes is performed under conditions (e.g., those described below for proliferation) that preserve lymphocyte viability. In one embodiment, the temperature, salinity and protein milieu permissive of cell growth is 37 °C, RPMI 1640 Medium with 10% fetal bovine serum ("FBS") and 1% penicillin ("PS"). In an unexpected feature of this invention, the lymphocytes (e.g., B lymphocytes) remain viable for an extended duration, thereby permitting lymphocyte PKCε measurement more than three hours after harvesting the lymphocytes. In one embodiment of this invention, the lymphocyte-culturing step is performed for more than three hours. Preferably, the lymphocyte-culturing step is performed for more than six hours (e.g., overnight). In immortalized B lymphocytes, the PKCε levels remain stable indefinitely and thus can be measured days, weeks, months or years after immortalization.

As used herein, culturing lymphocytes "from" a subject means culturing lymphocytes originating from the subject, wherein prior to culturing, the lymphocytes either have or have not been manipulated (e.g., isolated, immortalized and/or otherwise modified) following removal from the subject.

As used herein, "lymphocytes" include, by way of example, B lymphocytes, T lymphocytes, and mixtures thereof. Preferably, the lymphocytes are B lymphocytes. Methods for obtaining lymphocytes from a subject's blood are known, and include, for example, flow cytometry, Ficoll (a hydrophilic polysaccharide that separates layers of blood), and gradient centrifugation. Additionally, in the subject methods, the lymphocytes (e.g., B lymphocytes) can be used in immortalized or primary (i.e., non-immortalized) form. Methods for immortalizing lymphocytes (e.g., B lymphocytes) are known, and include, for example, treating the lymphocytes with Epstein-Barr virus ("EBV").

As used herein, "PKCε" (also referred to herein as "PKCe") shall mean protein kinase C epsilon.

As used herein, "measuring" the amount of PKCε in the cultured lymphocytes can be performed, for example, *in situ* or after isolating protein from the lymphocytes. Moreover, the measuring can be performed, for example, using any method by which the amount of a protein kinase can be quantitatively determined. Such methods are known and include, without limitation, ELISA-based assays, spectrophotometric assays, Western blot methods, Duolink methods, and any other antibody-based method. In one embodiment, measuring the amount of PKCε in cultured lymphocytes means determining the amount of PKCε as expressed, for example, in (i) fg of PKCε per lymphocyte, (ii) ng of PKCε per known population of cells, (iii) ng of PKCε per volume of cell digest, or (iv) ng of PKCε per volume of supernatant generated during the PKCε isolation process. In another embodiment, such measurements are expressed in relation to total lymphocyte protein. For example, the amount of PKCε measured in cultured lymphocytes can be expressed as "ng/mL/µg", wherein "mL" represents the volume of supernatant generated during the PKCε isolation process, "ng" represents the amount of PKCε present in the supernatant, and "µg" represents the amount of protein present in the supernatant. In a further embodiment, measuring the amount of PKCε in cultured lymphocytes means determining the rate of change over time in the amount of PKCε as expressed, for example, in one or more of the units described in this paragraph.

As used herein, a "suitable control" for performing the subject methods requiring same includes, without limitation, a positive control, a negative control, or one or more of each. For example, a positive control could be lymphocyte PKCε data obtained by performing one of the subject methods on lymphocytes obtained from a human subject afflicted with Alzheimer's disease. A negative control could be, for example, lymphocyte PKCε data obtained by performing one of the subject methods on lymphocytes obtained from a human subject who is not afflicted with Alzheimer's disease (e.g., a subject without any cognitive symptoms, a subject afflicted with mild cognitive impairment, or a subject afflicted with non-Alzheimer's dementia). Importantly, it is envisioned that in some of the subject methods, control tests (e.g., positive, negative and/or both) will be performed before, concurrently with or after testing the lymphocytes from the human subjects of interest. The data from such control tests can then serve as suitable controls. It is also envisioned that in other of the subject methods, no control tests are performed before, concurrently with or after testing the lymphocytes from the human subjects of interest. Instead, in each such method, determining affliction with, or predisposition to, Alzheimer's disease (as applicable) can be achieved, for example, by comparing the method's result with lymphocyte PKCε parameters (e.g., 1.0 ng/mL/pg) having a known correlation with disease state or predisposition, as applicable.

As used herein, an "amylospheroid" (also referred to herein as "ASPD") means a spherical aggregate of β-amyloid. Amylospheroids are known and commercially available. Preferably, the amylospheroid is of a toxic size, such as having a diameter of greater than 10 nm (e.g., 10 nm to 20 nm, 20 nm to 50 nm, and 50 nm to 100 nm).

As used herein, a "suitable concentration" of amylospheroid includes, without limitation, greater than 0 nM and less than 500 nM (e.g., 1 nM to 100 nM, 100 nM to 200 nM, 200 nM to 300 nM, 300 nM to 400 nM, and 400 nM to 500 nM).

As used herein, "separately measuring", with respect to the amount of PKCε in two different cultured lymphocyte populations, means measuring the amount of PKCε in each population without first mixing or otherwise combining the two populations.

### Embodiments of the Invention

This invention provides lymphocyte-based methods for diagnosing Alzheimer's disease in a human subject. It also provides lymphocyte-based methods for determining whether a human subject is predisposed to becoming afflicted with Alzheimer's disease. The subject methods are based, at least in part, on the surprising discovery that a subject's lymphocytes can be used in a PKCε-based test to either diagnose Alzheimer's disease or determine a predisposition to Alzheimer's disease, as applicable.

Specifically, disclosed but not claimed is a first method, namely, a method for diagnosing Alzheimer's disease in a symptomatic human subject comprising the steps of (a) culturing lymphocytes from the subject under conditions that preserve lymphocyte viability and/or permit the lymphocytes to proliferate; (b) measuring the amount of PKCε in the cultured lymphocytes; and (c) comparing the measurement of step (b) with a suitable control, thereby determining whether the subject is afflicted with Alzheimer's disease.

In this first method and the second, third, fourth, fifth and sixth methods described below, the lymphocytes can be any type of lymphocytes. Preferably, the lymphocytes are B lymphocytes. Also preferred are B lymphocytes that are immortalized.

In this first method and the second, third, fourth, fifth and sixth methods described below, wherein culturing step (a) is performed for more than three hours, and ideally for more than six hours.

In a preferred embodiment of this first method and the third and fifth methods described below, the symptomatic human subject is suspected of having either Alzheimer's disease or non-Alzheimer's dementia, and the method permits determining with which of these two disorders the subject is afflicted.

In a preferred embodiment of this first method and the second, third, fourth, fifth and sixth methods described below, this invention provides a method wherein step (a) comprises culturing immortalized B lymphocytes from the subject for more than six hours.

Disclosed but not claimed is a second method, namely, a method for determining whether a human subject is predisposed to becoming afflicted with Alzheimer's disease comprising the steps of (a) culturing lymphocytes from the subject under conditions that preserve lymphocyte viability and/or permit the lymphocytes to proliferate; (b) measuring the amount of PKCε in the cultured lymphocytes; and (c) comparing the measurement of step (b) with a suitable control, thereby determining whether the subject is predisposed to becoming afflicted with Alzheimer's disease.

In this second method and the fourth and sixth methods described below, the subject may be afflicted with a cognitive disability. In one embodiment, this disability is mild cognitive impairment. Alternatively, in this second method and the fourth and sixth methods described below, the subject is not cognitively impaired.

Disclosed but not claimed is a third method, namely, a method for diagnosing Alzheimer's disease in a symptomatic human subject comprising the steps of (a) culturing two otherwise identical populations of lymphocytes from the subject under conditions that preserve lymphocyte viability and/or permit the lymphocytes to proliferate, the first population comprising a suitable concentration of amylospheroid and the second population being free of amylospheroid; (b) separately measuring the amount of PKCε in the cultured lymphocytes from the first and second populations; and (c) comparing the measurements of step (b), whereby the subject is afflicted with Alzheimer's disease if the amount of PKCε in the cultured lymphocytes from the first population is greater than or equal to the amount of PKCε in the cultured lymphocytes from the second population.

Disclosed but not claimed is a fourth method, namely, a method for determining whether a human subject is predisposed to becoming afflicted with Alzheimer's disease comprising the steps of (a) culturing two otherwise identical populations of lymphocytes from the subject under conditions that preserve lymphocyte viability and/or permit the lymphocytes to proliferate, the first population comprising a suitable concentration of amylospheroid and the second population being free of amylospheroid; (b) separately measuring the amount of PKCε in the cultured lymphocytes from the first and second populations; and (c) comparing the measurements of step (b), whereby the subject is predisposed to becoming afflicted with Alzheimer's disease if the amount of PKCε in the cultured lymphocytes from the first population is greater than or equal to the amount of PKCε in the cultured lymphocytes from the second population.

Disclosed but not claimed is a fifth method, namely, a method for diagnosing Alzheimer's disease in a symptomatic human subject comprising the steps of (a) culturing two otherwise identical populations of lymphocytes from the subject under conditions that preserve lymphocyte viability and/or permit the lymphocytes to proliferate, the first population comprising a first suitable concentration of amylospheroid and the second population comprising a second suitable concentration of amylospheroid higher than the first concentration; (b) separately measuring the amount of PKCε in the cultured lymphocytes from the first and second populations; and (c) comparing the measurements of step (b), whereby the subject is afflicted with Alzheimer's disease if the amount of PKCε in the cultured lymphocytes from the second population is greater than the amount of PKCε in the cultured lymphocytes from the first population.

Disclosed but not claimed is a sixth method, namely, a method for determining whether a human subject is predisposed to becoming afflicted with Alzheimer's disease comprising the steps of (a) culturing two otherwise identical populations of lymphocytes from the subject under conditions that preserve lymphocyte viability and/or permit the lymphocytes to proliferate, the first population comprising a first suitable concentration of amylospheroid and the second population comprising a second suitable concentration of amylospheroid higher than the first concentration; (b) separately measuring the amount of PKCε in the cultured lymphocytes from the first and second populations; and (c) comparing the measurements of step (b), whereby the subject is predisposed to becoming afflicted with Alzheimer's disease if the amount of PKCε in the cultured lymphocytes from the second population is greater than the amount of PKCε in the cultured lymphocytes from the first population.

In the fifth and sixth methods, the first and second suitable concentrations of amylospheroid (again, represented by "ASPD") include, for example, values ranging from 0 nM to 500 nM, with the proviso that at least the second amylospheroid concentration is greater than 0 nM. Preferably, the first and second suitable concentrations of ASPD include values ranging from 0 nM to 125 nM, as exemplified by the following two-concentration permutations: (i) 0 nM and 25 nM; (ii) 0 nM and 50 nM; (iii) 0 nM and 75 nM; (iv) 0 nM and 100 nM; and (v) 0 nM and 125 nM.

In one embodiment of the fifth and sixth methods, the method comprises culturing more than two otherwise identical populations of lymphocytes from the subject, each population comprising a unique suitable concentration of ASPD, and separately measuring the amount of PKCε in the cultured lymphocytes from each population. Examples of first, second and third suitable concentrations of ASPD, to name just one embodiment, include the following: (i) 0 nM, 25 nM and 50 nM; (ii) 0 nM, 50 nM and 100 nM; and (iii) 0 nM, 50 nM and 125 nM.

The diagnostic outcome of the fifth method, and the prognostic outcome of the sixth method, can be expressed, in one embodiment, by plotting the PKCε measurements (i.e., data points) on a graph and measuring the slope of the line formed by the data points. As a specific example, when the PKCε measurements are plotted on a graph wherein the x-axis = ASPD concentration (nM) (e.g., 0 nM to 100 nM, 125 nM, 200 nM, 300 nM, 400 nM or 500 nM) and the y-axis = PKCε (ng/mL/µg of protein), the resulting slope is positive (e.g., greater than 0.000, greater than 0.005, or greater than 0.010). Preferably, each data point plotted is an averaged value.

Finally, disclosed but not claimed is a kit for performing any of the subject methods, wherein the kit comprises, in separate compartments or a single compartment, (i) a growth medium (e.g., RPMI 1640 Medium with 10% FBS and 1% PS) and (ii) a means for quantitatively measuring PKCε (e.g., a PKCε ELISA kit with sandwich human antibody). The kit may optionally contain ASPD suitably formulated for performing the subject ASPD-dependent methods. PKCε ELISA kits are commercially available (e.g., https://www.mybiosource.com/prods/ELISA-Kit/Human/Protein-Kinase-C-Epsilon/PKCE/datasheet.php?products_id=21356 or http://www.antibodies-online.com/search.php).

Also envisioned is the application of the instant invention to (i) any non-lymphocyte peripheral blood mononuclear cell (PBMC) and (ii) any pluripotent stem cell (iPSC) derived from a reprogrammed EBV-immortalized B lymphocyte (https://www.ncbi.nlm.nih.gov/pmc/articles/PMC3158714/; and https://www.ncbi.nlm.nih.gov/pmc/articles/PMC2596471/). In this embodiment, such cells are treated, mutatis mutandis, as lymphocytes are treated in this invention.

This invention will be better understood by reference to the examples which follow, but those skilled in the art will readily appreciate that the specific examples detailed are only illustrative of the invention as described more fully in the claims which follow thereafter.

### Example 1

PBMC Preparation. Whole blood, sampled in BD Vacutainer CPTTM Cell Preparation Tubes with sodium heparin (BD Bioscience, Franklin Lakes, NJ, USA), was diluted 1:2 in PBS and gently layered over cold Ficoll-Paque PLUS (GE Healthcare, Little Chalfont, UK) and kept on ice before centrifugation for 20 min at 900 × g without brake at 18-20 °C. The cell layer on top of the Ficoll-Paque PLUS consisting of peripheral blood mononuclear cells (PBMCs) was collected and diluted in PBS. PBMCs were centrifuged again for 7 min at 450 × g at 18-20 °C. The supernatant was removed and cells were re-suspended in 5 ml PBS and counted before they were centrifuged at 350 × g for seven min at 18-20 °C. Finally, cells were re-suspended in freezing medium (10% DMSO in heat-inactivated fetal bovine serum (FBS) (Nordic Biolabs AB, Täby, Sweden) and placed at -70°C in a Mr. Frosty^{™} Freezing Container (Thermo Fisher) for at least three hours before being transferred to liquid nitrogen for extended storage.

Flow Cytometry. For each individual cell sample, 5 × 10⁶ PBMCs were quickly thawed at 37 °C and diluted in 40 ml cold wash buffer (PBS supplemented with 2.5% FBS (Life Technologies Ltd. Paisley, UK) and 0.1% sodium azide). The cell suspension was centrifuged at 250 × g for five min and after discarding the supernatant, the pellet was re-suspended in 400 ml wash buffer. Each sample was stained for one hour in a light-protected environment at 2 °C with titrated amounts of anti-CD1 9 labeled with Alexa Fluor 700 (BD Biosciences). Samples were analyzed using a BD LSR II Special Order System, controlled by the BD FACSDiva 6.0 software (BD Biosciences). A preliminary forward scatter (FSC) versus side scatter (SSC) gate was used to identify lymphocytes and, depending on sample size, a total of up to 100,000 in-gate events were recorded. All datasets were migrated to FlowJo 7.6.5 (Treestar Inc. Ashland, OR, USA) for further gating and analysis. Cells were cultured as per Coriell Cell Repository specifications, i.e., at 37 °C, in water jacket CO₂ incubators in RPM I 1640 Medium, with 10% FBS and 1% PS.

## Claims

1. A method for diagnosing Alzheimer's disease in a symptomatic human subject comprising the steps of (a) culturing two otherwise identical populations of B lymphocytes from the subject under conditions that preserve B lymphocyte viability and/or permit the B lymphocytes to proliferate, the first population comprising a suitable concentration of amylospheroid of greater than 0 nM and less than 500 nM and the second population being free of amylospheroid; (b) separately measuring the amount of PKCε in the cultured B lymphocytes from the first and second populations; and (c) comparing the measurements of step (b), whereby the subject is afflicted with Alzheimer's disease if the amount of PKCε in the cultured B lymphocytes from the first population is greater than or equal to the amount of PKCε in the cultured B lymphocytes from the second population.

2. A method for determining whether a human subject is predisposed to becoming afflicted with Alzheimer's disease comprising the steps of (a) culturing two otherwise identical populations of B lymphocytes from the subject under conditions that preserve B lymphocyte viability and/or permit the B lymphocytes to proliferate, the first population comprising a suitable concentration of amylospheroid of greater than 0 nM and less than 500 nM and the second population being free of amylospheroid; (b) separately measuring the amount of PKCε in the cultured B lymphocytes from the first and second populations; and (c) comparing the measurements of step (b), whereby the subject is predisposed to becoming afflicted with Alzheimer's disease if the amount of PKCε in the cultured B lymphocytes from the first population is greater than or equal to the amount of PKCε in the cultured B lymphocytes from the second population.

3. A method for diagnosing Alzheimer's disease in a symptomatic human subject comprising the steps of (a) culturing two otherwise identical populations of Blymphocytes from the subject under conditions that preserve B lymphocyte viability and/or permit the B lymphocytes to proliferate, the first population comprising a first suitable concentration of amylospheroid of greater than 0 nM and less than 500 nM and the second population comprising a second suitable concentration of amylospheroid of greater than 0 nM and less than 500 nM, the second concentration being higher than the first concentration; (b) separately measuring the amount of PKCε in the cultured B lymphocytes from the first and second populations; and (c) comparing the measurements of step (b), whereby the subject is afflicted with Alzheimer's disease if the amount of PKCε in the cultured B lymphocytes from the second population is greater than the amount of PKCε in the cultured B lymphocytes from the first population.

4. A method for diagnosing Alzheimer's disease in a symptomatic human subject comprising the steps of (a) culturing two otherwise identical populations of B lymphocytes from the subject under conditions that preserve B lymphocyte viability and/or permit the B lymphocytes to proliferate, the first population comprising a first suitable concentration of amylospheroid of greater than 0 nM and less than 500 nM, and the second population comprising a second suitable concentration of amylospheroid of greater than 0 nM and less than 500 nM, wherein the second concentration is higher than the first concentration; (b) separately measuring the amount of PKCε in the cultured B lymphocytes from the first and second populations; and (c) comparing the measurements of step (b), whereby the subject is afflicted with Alzheimer's disease if when the PKCε measurements are plotted on a graph wherein the x-axis shows amylospheroid concentration in nM and the y-axis shows PKCε in ng/mL/µg of protein, the resulting slope is greater than 0.000.

5. The method of claim 4, wherein the resulting slope is greater than 0.005

6. The method of claim 4, wherein the resulting slope is greater than 0.010.

7. The method of any of the claims 1, 3 to 6, wherein the symptomatic human subject is suspected of having either Alzheimer's disease or non-Alzheimer's dementia, and the method permits determining with which of these two disorders the subject is afflicted.

8. A method for determining whether a human subject is predisposed to becoming afflicted with Alzheimer's disease comprising the steps of (a) culturing two otherwise identical populations of B lymphocytes from the subject under conditions that preserve B lymphocyte viability and/or permit the B lymphocytes to proliferate, the first population comprising a first suitable concentration of amylospheroid of greater than 0 nM and less than 500 nM, and the second population comprising a second suitable concentration of amylospheroid of greater than 0 nM and less than 500 nM, wherein the second concentration is higher than the first concentration; (b) separately measuring the amount of PKCε in the cultured B lymphocytes from the first and second populations; and (c) comparing the measurements of step (b), whereby the subject is predisposed to becoming afflicted with Alzheimer's disease if the amount of PKCε in the cultured B lymphocytes from the second population is greater than the amount of PKCε in the cultured B lymphocytes from the first population.

9. The method of any of the claims 1 to 8, wherein the subject is afflicted with mild cognitive impairment.

10. The method of any of the claims 1 to 8, wherein the subject is not cognitively impaired.

11. The method of any of the claims 1 to 10, wherein the B lymphocytes are immortalized.

12. The method of any of the claims 1 to 11, wherein step (a) is performed for more than three hours.

13. The method of claim 11, wherein step (a) comprises culturing immortalized B lymphocytes from the subject for more than six hours.

## Patentansprüche

1. Verfahren zur Diagnose von Alzheimer-Krankheit bei einem symptomatischen menschlichen Subjekt, umfassend die Schritte: (a) Kultivieren von zwei ansonsten identischen B-Lymphozyten-Populationen des Subjekts unter Bedingungen, die die B-Lymphozyten-Viabilität erhalten und/oder die B-Lymphozyten-Proliferation ermöglichen, wobei die erste Population eine geeignete Amylosphäroid-Konzentration von mehr als 0 nM und weniger als 500 nM umfasst und die zweite Population frei von Amylosphäroid ist; (b) separat Messen der PKC_{ε}-Menge in den kultivierten B-Lymphozyten aus den ersten und zweiten Populationen; und (c) Vergleichen der Messungen von Schritt (b), wobei das Subjekt an Alzheimer erkrankt ist, wenn die PKC_{ε}-Menge in den kultivierten B-Lymphozyten aus der erste Population größer als die PKC_{ε}-Menge oder gleich der PKC_{ε}-Menge in den kultivierten B-Lymphozyten aus der zweiten Population ist.

2. Verfahren, um festzustellen, ob ein menschliches Subjekt prädisponiert ist, an Alzheimer zu erkranken, umfassend die Schritte: (a) Kultivieren von zwei ansonsten identischen B-Lymphozyten-Populationen des Subjekts unter Bedingungen, die die B-Lymphozyten-Viabilität erhalten und/oder die B-Lymphozyten-Proliferation ermöglichen, wobei die erste Population eine geeignete Amylosphäroid-Konzentration von mehr als 0 nM und weniger als 500 nM umfasst und die zweite Population frei von Amylosphäroid ist; (b) separat Messen der PKC_{ε}-Menge in den kultivierten B-Lymphozyten aus den ersten und zweiten Populationen; und (c) Vergleichen der Messungen von Schritt (b), wobei das Subjekt prädisponiert ist, an Alzheimer zu erkranken, wenn die PKC_{ε}-Menge in den kultivierten B-Lymphozyten aus der erste Population größer als die PKC_{ε}-Menge oder gleich der PKC_{ε}-Menge in den kultivierten B-Lymphozyten aus der zweiten Population ist.

3. Verfahren zur Diagnose von Alzheimer-Krankheit bei einem symptomatischen menschlichen Subjekt, umfassend die Schritte: (a) Kultivieren von zwei ansonsten identischen B-Lymphozyten-Populationen des Subjekts unter Bedingungen, die die B-Lymphozyten-Viabilität erhalten und/oder die B-Lymphozyten-Proliferation ermöglichen, wobei die erste Population eine erste geeignete Amylosphäroid-Konzentration von mehr als 0 nM und weniger als 500 nM umfasst und die zweite Population eine zweite geeignete Amylosphäroid-Konzentration von mehr als 0 nM und weniger als 500 nM umfasst, wobei die zweite Konzentration höher als die erste Konzentration ist; (b) separat Messen der PKC_{ε}-Menge in den kultivierten B-Lymphozyten aus den ersten und zweiten Populationen; und (c) Vergleichen der Messungen von Schritt (b), wobei das Subjekt an Alzheimer erkrankt ist, wenn die PKC_{ε}-Menge in den kultivierten B-Lymphozyten aus der zweiten Population größer als die PKC_{ε}-Menge in den kultivierten B-Lymphozyten aus der ersten Population ist.

4. Verfahren zur Diagnose von Alzheimer-Krankheit bei einem symptomatischen menschlichen Subjekt, umfassend die Schritte: (a) Kultivieren von zwei ansonsten identischen B-Lymphozyten-Populationen des Subjekts unter Bedingungen, die die B-Lymphozyten-Viabilität erhalten und/oder die B-Lymphozyten-Proliferation ermöglichen, wobei die erste Population eine erste geeignete Amylosphäroid-Konzentration von mehr als 0 nM und weniger als 500 nM umfasst und die zweite Population eine zweite geeignete Amylosphäroid-Konzentration von mehr als 0 nM und weniger als 500 nM umfasst, wobei die zweite Konzentration höher als die erste Konzentration ist; (b) separat Messen der PKC_{ε}-Menge in den kultivierten B-Lymphozyten aus den ersten und zweiten Populationen; und (c) Vergleichen der Messungen von Schritt (b), wobei das Subjekt an Alzheimer erkrankt ist, wenn, wenn die PKC_{ε}-Messungen in einen Graphen übertragen werden, bei dem die x-Achse die Amylosphäroid-Konzentration in nM wiedergibt und die y-Achse den PKC_{ε}-Wert in ng/ml/pg Protein wiedergibt, die resultierende Steigung größer als 0,000 ist.

5. Verfahren nach Anspruch 4, wobei die resultierende Steigung größer als 0,005 ist.

6. Verfahren nach Anspruch 4, wobei die resultierende Steigung größer als 0,010 ist.

7. Verfahren nach einem der Ansprüche 1, 3 bis 6, bei dem vermutet wird, dass das symptomatische menschliche Subjekt entweder mit Alzheimer-Krankheit oder mit Nicht-Alzheimer-Demenz behaftet ist, und wobei das Verfahren es erlaubt, festzustellen, mit welcher dieser beiden Störungen das Subjekt behaftet ist.

8. Verfahren, um festzustellen, ob ein menschliches Subjekt prädisponiert ist, an Alzheimer zu erkranken, umfassend die Schritte: (a) Kultivieren von zwei ansonsten identischen B-Lymphozyten-Populationen des Subjekts unter Bedingungen, die die B-Lymphozyten-Viabilität erhalten und/oder die B-Lymphozyten-Proliferation ermöglichen, wobei die erste Population eine erste geeignete Amylosphäroid-Konzentration von mehr als 0 nM und weniger als 500 nM umfasst und die zweite Population eine zweite geeignete Amylosphäroid-Konzentration von mehr als 0 nM und weniger als 500 nM umfasst, wobei die zweite Konzentration höher als die erste Konzentration ist; (b) separat Messen der PKC_{ε}-Menge in den kultivierten B-Lymphozyten aus den ersten und zweiten Populationen; und (c) Vergleichen der Messungen von Schritt (b), wobei das Subjekt prädisponiert ist, an Alzheimer zu erkranken, wenn die PKC_{ε}-Menge in den kultivierten B-Lymphozyten aus der zweiten Population größer als die PKC_{ε}-Menge in den kultivierten B-Lymphozyten aus der ersten Population ist.

9. Verfahren nach einem der Ansprüche 1 bis 8, wobei das Subjekt an leichter kognitiver Beeinträchtigung leidet.

10. Verfahren nach einem der Ansprüche 1 bis 8, wobei das Subjekt nicht kognitiv beeinträchtigt ist.

11. Verfahren nach einem der Ansprüche 1 bis 10, wobei die B-Lymphozyten immortalisiert sind.

12. Verfahren nach einem der Ansprüche 1 bis 11, wobei Schritt (a) mehr als drei Stunden lang durchgeführt wird.

13. Verfahren nach Anspruch 11, wobei Schritt (a) das mehr als sechsstündige Kultivieren immortalisierter B-Lymphozyten des Subjekts umfasst.

## Revendications

1. Méthode permettant de diagnostiquer la maladie d'Alzheimer chez un sujet humain symptomatique, comprenant les étapes suivantes : (a) culture de deux populations de lymphocytes B sinon identiques obtenus du sujet, dans des conditions qui préservent la viabilité des lymphocytes B et/ou permettent la prolifération des lymphocytes B, la première population comprenant des amylosphéroïdes à une concentration appropriée supérieure à 0 nM et inférieure à 500 nM et la seconde population étant exempte d'amylosphéroïdes ; (b) mesures séparées de la quantité de PKCε dans les lymphocytes B en culture dérivés des première et seconde populations ; et (c) comparaison des mesures de l'étape (b), où le sujet est atteint de la maladie d'Alzheimer si la quantité de PKCε dans les lymphocytes B en culture dérivés de la première population est supérieure ou égale à la quantité de PKCε dans les lymphocytes B en culture dérivés de la seconde population.

2. Méthode permettant de déterminer si un sujet humain est prédisposé à développer la maladie d'Alzheimer, comprenant les étapes suivantes : (a) culture de deux populations de lymphocytes B sinon identiques obtenus du sujet, dans des conditions qui préservent la viabilité des lymphocytes B et/ou permettent la prolifération des lymphocytes B, la première population comprenant des amylosphéroïdes à une concentration appropriée supérieure à 0 nM et inférieure à 500 nM et la seconde population étant exempte d'amylosphéroïdes ; (b) mesures séparées de la quantité de PKCε dans les lymphocytes B en culture dérivés des première et seconde populations ; et (c) comparaison des mesures de l'étape (b), où le sujet est prédisposé à développer la maladie d'Alzheimer si la quantité de PKCε dans les lymphocytes B en culture dérivés de la première population est supérieure ou égale à la quantité de PKCε dans les lymphocytes B en culture dérivés de la seconde population.

3. Méthode permettant de diagnostiquer la maladie d'Alzheimer chez un sujet humain symptomatique, comprenant les étapes suivantes : (a) culture de deux populations de lymphocytes B sinon identiques obtenus du sujet, dans des conditions qui préservent la viabilité des lymphocytes B et/ou permettent la prolifération des lymphocytes B, la première population comprenant des amylosphéroïdes à une première concentration appropriée supérieure à 0 nM et inférieure à 500 nM et la seconde population comprenant des amylosphéroïdes à une seconde concentration appropriée supérieure à 0 nM et inférieure à 500 nM, la seconde concentration étant plus élevée que la première concentration ; (b) mesures séparées de la quantité de PKCε dans les lymphocytes B en culture dérivés des première et seconde populations ; et (c) comparaison des mesures de l'étape (b), où le sujet est atteint de la maladie d'Alzheimer si la quantité de PKCε dans les lymphocytes B en culture dérivés de la seconde population est supérieure à la quantité de PKCε dans les lymphocytes B en culture dérivés de la première population.

4. Méthode permettant de diagnostiquer la maladie d'Alzheimer chez un sujet humain symptomatique, comprenant les étapes suivantes : (a) culture de deux populations de lymphocytes B sinon identiques obtenus du sujet, dans des conditions qui préservent la viabilité des lymphocytes B et/ou permettent la prolifération des lymphocytes B, la première population comprenant des amylosphéroïdes à une première concentration appropriée supérieure à 0 nM et inférieure à 500 nM et la seconde population comprenant des amylosphéroïdes à une seconde concentration appropriée supérieure à 0 nM et inférieure à 500 nM, la seconde concentration étant plus élevée que la première concentration ; (b) mesures séparées de la quantité de PKCε dans les lymphocytes B en culture dérivés des première et seconde populations ; et (c) comparaison des mesures de l'étape (b), où le sujet est atteint de la maladie d'Alzheimer si, quand les mesures de la PKCε sont portées sur une courbe où l'axe des x représente la concentration en amylosphéroïdes en nM et l'axe des y représente la PKCε en ng/ml/µg de protéines, la pente générée est supérieure à 0,000.

5. Méthode de la revendication 4, où la pente générée est supérieure à 0,005.

6. Méthode de la revendication 4, où la pente générée est supérieure à 0,010.

7. Méthode de l'une quelconque des revendications 1, 3 à 6, où le sujet humain symptomatique est suspecté de présenter la maladie d'Alzheimer ou une démence de type non Alzheimer, et où la méthode permet de déterminer de laquelle de ces deux affections le sujet est atteint.

8. Méthode permettant de déterminer si un sujet humain est prédisposé à développer la maladie d'Alzheimer, comprenant les étapes suivantes : (a) culture de deux populations de lymphocytes B sinon identiques obtenus du sujet, dans des conditions qui préservent la viabilité des lymphocytes B et/ou permettent la prolifération des lymphocytes B, la première population comprenant des amylosphéroïdes à une première concentration appropriée supérieure à 0 nM et inférieure à 500 nM et la seconde population comprenant des amylosphéroïdes à une seconde concentration appropriée supérieure à 0 nM et inférieure à 500 nM, la seconde concentration étant plus élevée que la première concentration ; (b) mesures séparées de la quantité de PKCε dans les lymphocytes B en culture dérivés des première et seconde populations ; et (c) comparaison des mesures de l'étape (b), où le sujet est prédisposé à développer la maladie d'Alzheimer si la quantité de PKCε dans les lymphocytes B en culture dérivés de la seconde population est supérieure à la quantité de PKCε dans les lymphocytes B en culture dérivés de la première population.

9. Méthode de l'une quelconque des revendications 1 à 8, où le sujet présente un trouble cognitif léger.

10. Méthode de l'une quelconque des revendications 1 à 8, où le sujet ne présente aucun trouble cognitif.

11. Méthode de l'une quelconque des revendications 1 à 10, où les lymphocytes B sont immortalisés.

12. Méthode de l'une quelconque des revendications 1 à 11, où l'étape (a) est effectuée pendant plus de trois heures.

13. Méthode de la revendication 11, où l'étape (a) comprend la culture, pendant plus de six heures, de lymphocytes B obtenus du sujet immortalisés.
